# FASCICULE DE BREVET EUROPEEN

(11) **EP 3 737 254 B1**
(45) Date de publication et mention de la délivrance du brevet: **27.12.2023**
(21) Numéro de dépôt: 19703156.0
(22) Date de dépôt: 07.01.2019
(51) Int. Cl.: A43B 3/00, A61B 5/11, G08B 21/04

(54) **CHAUSSURE COMPRENANT UN ACCÉLÉROMÈTRE, ENSEMBLE ET PROCÉDÉ DE DÉTECTION DE CHUTE CORRESPONDANT**
SCHUH MIT EINEM BESCHLEUNIGUNGSMESSER, ANORDNUNG UND ENTSPRECHENDES FALLERFASSUNGSVERFAHREN
BOOT COMPRISING AN ACCELEROMETER, ASSEMBLY AND CORRESPONDING FALL DETECTION METHOD

(30) Priorité: 08.01.2018 FR 1850127
(43) Date de publication de la demande: 18.11.2020
(73) Titulaire: PARADE, 49111 Montrevault-sur-Èvre (FR)
(72) Inventeur: FORTUMEAU, Julien, 44115 HAUTE GOULAINE (FR); BRISSEAU, Joslain, 49800 TRELAZE (FR); LEBLON, Laure, 44115 BASSE GOULAINE (FR); CHEREL, Franck, 49460 MONTREUIL JUIGNE (FR)
(74) Mandataire: Ipsilon
(86) Numéro de dépôt international: PCT/FR2019/050030
(87) Numéro de publication internationale: WO 2019/135059

(56) Documents cités:
- WO-A1-2016/120703
- CN-A- 104 821 061
- US-A1- 2012 101 770
- US-A1- 2016 157 756

## Description

### DOMAINE DE L'INVENTION

La présente invention concerne de manière générale la détection de chute.

### ART ANTERIEUR

L'invention concerne plus particulièrement une chaussure et un ensemble de chaussures permettant de détecter une chute d'une personne (porteur) munie de la ou des chaussures.

On connait de l'état de la technique, et notamment du document EP2774502, des chaussures munies d'un accéléromètre et d'un gyroscope dont les données sont utilisées pour tenter de détecter la chute d'une personne. On connait également des documents US2016157756, US2012101770, CN104464189, US2013185003, US2015157274, WO2016/120703, et CN104821061 d'autres systèmes utilisés pour analyser la démarche d'une personne ou tenter de détecter la chute d'une personne.

Cependant, il est souhaitable de pouvoir améliorer la fiabilité de détection de chute.

La présente invention a pour but de proposer une nouvelle chaussure, un nouvel ensemble de chaussures et un nouveau procédé correspondant permettant de palier tout ou partie des problèmes exposés ci-dessus.

### RESUME DE L'INVENTION

A cet effet, l'invention a pour objet une chaussure comprenant un accéléromètre permettant de fournir des données d'accélération selon trois axes distincts X, Y, Z, l'axe Z étant orienté verticalement lorsque la chaussure est posée à plat (c'est-à-dire debout) sur un sol horizontal,
ladite chaussure comportant aussi un système de pilotage qui comprend :
   - une mémoire permettant d'enregistrer les données fournies par l'accéléromètre;
   - une unité de traitement de données, telle qu'un processeur,
caractérisée en ce que l'unité de traitement est configurée pour réaliser les étapes suivantes :
   - comparer l'accélération mesurée par l'accéléromètre selon au moins un axe, avec une valeur seuil, telle que 6g, et,
   - en fonction au moins du résultat de la comparaison, déclencher la détermination des paramètres suivants :
      - la pente de la courbe, de préférence filtrée, de l'accélération mesurée selon l'axe Z en fonction du temps,
      - la durée, dite durée de position horizontale, pendant laquelle l'accélération ou la variation d'accélération selon un axe, ou plusieurs des axes de l'accéléromètre, reste inférieure à une valeur seuil ;
   - détecter une chute au moins en fonction de ladite pente et de ladite durée de position horizontale,
ledit module de traitement étant configuré pour, en cas de détection de chute, générer un signal correspondant de détection de chute ;
et en ce que le système de pilotage comprend aussi un module de communication radio permettant de transmettre ledit signal de détection de chute à un récepteur distant.

Une telle conception de chaussure permet d'améliorer la fiabilité de détection d'une chute accidentelle d'une personne, telle qu'une personne âgée ou un travailleur isolé.

La personne munie d'une telle chaussure bénéficie ainsi d'un système électronique permettant de détecter la perte de verticalité et si besoin de lancer une procédure d'alerte vers un dispositif récepteur, par ondes radio, par exemple par un réseau de communication du type de l'Internet des objets ou encore un réseau de type LPWAN, c'est-à-dire à longue portée et à faible consommation d'énergie.

Selon un aspect particulier, la chaussure inclut aussi une source d'énergie, telle qu'une batterie, permettant d'alimenter les différents composants électroniques inclus dans la chaussure. Dans le cas d'un ensemble de deux chaussures communiquant entre elles comme présenté ci-après, chaque chaussure comprend une source d'énergie, telle qu'une batterie, pour alimenter les composants électroniques correspondant inclus dans la chaussure.

La ou chaque chaussure peut aussi comporter une ou plusieurs des caractéristiques suivantes prises dans toute combinaison techniquement admissible.

Selon une caractéristique avantageuse de l'invention, l'unité de traitement est configurée pour réaliser ladite détermination de paramètres à partir des données enregistrées sur une fenêtre de temps donnée, ladite fenêtre étant définie par une première durée donnée avant l'instant auquel l'unité de traitement détermine que ladite accélération mesurée est supérieure à ladite valeur seuil donnée, et par une deuxième durée donnée après cet instant.

Selon une caractéristique avantageuse de l'invention, l'unité de traitement est configurée pour déterminer ladite durée de position horizontale sur une partie de ladite fenêtre de temps dont l'instant de début est défini en fonction d'au moins l'un des paramètres suivants :
- la pente déterminée,
- l'instant auquel l'unité de traitement détermine que l'accélération mesurée est supérieure à ladite valeur seuil donnée.

Selon une caractéristique avantageuse de l'invention, l'unité de traitement est aussi configurée pour déterminer pour au moins un, de préférence pour chaque, axe de l'accéléromètre, une valeur d'accélération maximale, et pour détecter la chute en fonction aussi de ladite valeur d'accélération maximale.

Selon une caractéristique avantageuse de l'invention, l'unité de de traitement est aussi configurée pour déterminer un nombre de chocs, un choc correspondant à une accélération selon au moins l'un des axes de l'accéléromètre, supérieure à une valeur seuil données, telle que 3g, et pour détecter la chute en fonction aussi dudit nombre de chocs.

Selon une caractéristique avantageuse de l'invention, l'unité de traitement est aussi configurée pour déterminer l'orientation de la chaussure par rapport à la verticale, et pour détecter la chute en fonction aussi de ladite orientation de la chaussure.

Selon une caractéristique avantageuse de l'invention, la chaussure comprend un gyroscope permettant de fournir des données de giration au moins autour de l'axe X et/ou Y, et l'unité de traitement est configurée pour détecter la chute en fonction aussi des données de giration.

Selon une caractéristique avantageuse de l'invention, la chaussure comprend un capteur de détection de la présence d'un pied dans la chaussure.

Selon une caractéristique avantageuse de l'invention, le capteur est un capteur capacitif, de préférence logé dans un talon de la chaussure.

Selon une caractéristique avantageuse de l'invention, l'unité de traitement comprend aussi un module de vérification configuré pour détecter la présence d'un pied d'une personne dans la chaussure, et le déclenchement de la détermination de paramètres est aussi fonction du résultat de la détection de présence de pied dans la chaussure.

Selon une caractéristique avantageuse de l'invention, la chaussure présente un talon dans lequel l'accéléromètre est logé. On peut prévoir que, lorsque le gyroscope est présent, celui-ci est aussi logé dans le talon.

Selon une caractéristique avantageuse de l'invention, le module de traitement est configuré pour :
- calculer une valeur, appelée note, par axe pour plusieurs axes, de préférence pour chaque axe, en fonction desdits paramètres déterminés,
- de préférence, pondérer ladite note en fonction du poids et/ou de la taille de la personne,
- sommer les notes, de préférence pondérées, associées à chacun desdits axes et,
- détecter que ladite somme correspond à une chute lorsque ladite somme est supérieure à une valeur seuil prédéfinie.

Selon une caractéristique avantageuse de l'invention, ledit module de communication radio est configuré pour communiquer selon au moins l'un des protocoles suivants : LORA ; Nb-iot ; LTE-M.

Selon une caractéristique avantageuse de l'invention, la chaussure comprend un module de géolocalisation raccordé de manière directe ou indirecte au système de pilotage pour permettre d'acquérir la position géographique de la chaussure et transmettre cette position par ledit module de communication à un récepteur distant.

Selon une caractéristique avantageuse de l'invention, la chaussure comprend aussi un vibreur, de préférence inclus dans un talon de la chaussure, et raccordé de manière directe ou indirecte au système de pilotage.

L'invention concerne également un ensemble comprenant une chaussure telle que décrite ci-dessus, et une deuxième chaussure comprenant un accéléromètre de préférence inclus dans un talon de la deuxième chaussure, dans lequel chacune des première et deuxième chaussures comprend un module de communication radio, de préférence selon le protocole Bluetooth, lesdits modules de communication étant aptes à communiquer entre eux pour transmettre les données d'accéléromètre, depuis le module de communication de la deuxième chaussure au module de traitement de la première chaussure.

On peut aussi prévoir que la deuxième chaussure inclut aussi un gyroscope logé de préférence dans le talon et que les données du gyroscope puissent être transmises depuis le module de communication de la deuxième chaussure au module de traitement de la première chaussure.

Selon un aspect particulier, les accéléromètres sont agencés de la même manière dans la première chaussure et dans la deuxième chaussure. On peut aussi prévoir que les gyroscopes sont aussi agencés de la même manière dans la première chaussure et dans la deuxième chaussure.

L'invention concerne également un procédé de détection de chute à l'aide d'une chaussure ou d'un ensemble de chaussures tel que décrit(e) ci-dessus, dans lequel le procédé de détection de chute comprend les étapes suivantes :
- comparer l'accélération mesurée par l'accéléromètre selon au moins un axe, avec une valeur seuil, et,
- en fonction au moins du résultat de la comparaison, déclencher un procédé de détermination de paramètres qui comprend les étapes suivantes :
   - détermination de la pente de la courbe, de préférence filtrée, de l'accélération mesurée selon l'axe Z en fonction du temps,
   - détermination de la durée, dite durée de position horizontale, pendant laquelle l'accélération ou la variation d'accélération selon un axe ou plusieurs des axes de l'accéléromètre, reste inférieure à une valeur seuil ;
- détecter une chute au moins en fonction de ladite pente et de ladite durée de position horizontale,
et le procédé comprenant la génération d'un signal correspondant de détection de chute, et la transmission dudit signal de détection de chute à un récepteur distant.

L'invention concerne également un produit de programme informatique non transitoire comprenant des instructions de code de programme pour l'exécution des étapes du procédé de détection de chute tel que décrit ci-dessus, à l'aide d'une chaussure telle que décrite ci-dessus ou d'un ensemble de chaussures tel que décrit ci-dessus, lorsque ledit programme est exécuté par un processeur de la ou d'une des chaussures.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
- la figure 1 est une vue d'une chaussure conformément à un mode de réalisation de l'invention ;
- la figure 2 est une vue conformément à un mode de réalisation de l'invention d'une courbe d'accélération mesurée selon l'axe Z d'un accéléromètre présent dans la chaussure, à laquelle est superposée une autre courbe correspondant à ladite courbe d'accélération à l'état filtré ;
- la figure 3 est un graphique correspondant à un cas de chute d'une personne qui a trébuché en avançant, le graphique comprenant une courbe d'accélération mesurée selon chaque axe X, Y, Z de l'accéléromètre et une courbe filtrée d'accélération mesurée selon l'axe Z ;
- la figure 4 est une vue schématique conformément à un mode de réalisation de l'invention d'une première chaussure ;
- la figure 5 est une représentation schématique d'un système de pilotage pour un ensemble de chaussures conforme à un mode de réalisation de l'invention, une partie des composants du système de pilotage étant destinée à être répartie dans une chaussure et l'autre partie dans l'autre chaussure ;
- la figure 6 est une vue schématique conformément à un mode de réalisation de l'invention d'une deuxième chaussure apte à communiquer avec une première chaussure telle qu'illustrée à la figure 4 ;
- la figure 7 est un logigramme illustrant des étapes d'un mode de réalisation d'un procédé selon l'invention.

### DESCRIPTION DETAILLEE

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en oeuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. Les modes de réalisation qui suivent sont examinés, par souci de simplification, en relation avec la terminologie et la structure d'une chaussure et d'un système électronique.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

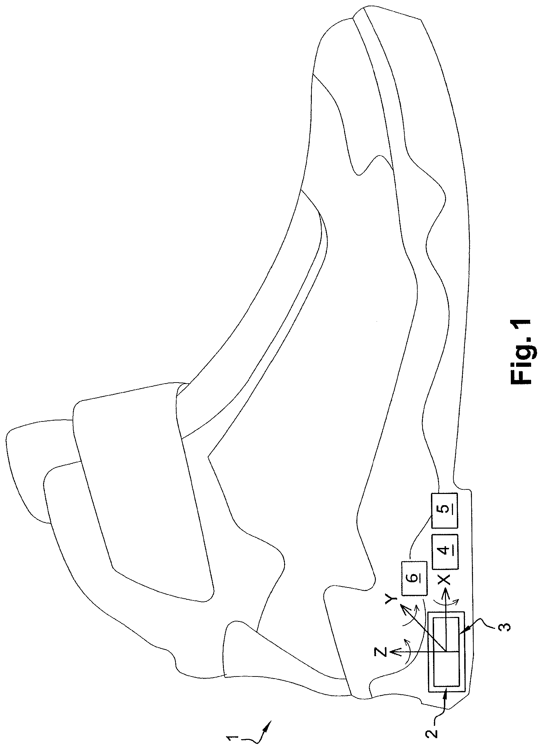

La figure 1 illustre une chaussure 1 qui présente un talon 10 dans lequel est logé un accéléromètre 2. Lorsque la chaussure 1 est posée à plat sur un sol horizontal, l'accéléromètre présente un axe Z vertical, dit axe de gravité, et deux axes X et Y horizontaux. L'accéléromètre 2, qui est solidaire de la chaussure 1, permet de fournir des données d'accélération AccX, AccY, AccZ respectivement selon les trois axes X, Y, Z. Les accélérations mesurées peuvent être considérées comme celles de la chaussure à laquelle l'accéléromètre est associé.

Selon un mode de réalisation particulier, le talon 10 loge aussi un gyroscope 3 permettant de fournir des données de giration, par exemple des angles de rotation ou des vitesses angulaires, autour des axes X et Y, et éventuellement Z. Les données de giration mesurées peuvent être considérées comme celles de la chaussure à laquelle le gyroscope est associé. Selon une variante de réalisation, on peut prévoir de ne pas utiliser de gyroscope. La suite de la description peut ainsi s'appliquer aussi au cas où le gyroscope n'est pas présent.

On peut prévoir que l'accéléromètre et le gyroscope soient intégrés au sein d'un même composant électronique. Ce composant électronique peut lui-même être logé avec d'autres composants, tels qu'une batterie dans un boîtier comme expliqué ci-après.

Ladite chaussure 1 comporte aussi un système de pilotage 4. Le système de pilotage se présente par exemple sous la forme d'un processeur et d'une mémoire de données dans laquelle sont stockées des instructions informatiques exécutables par ledit processeur, ou encore sous la forme d'un microcontrôleur.

Autrement dit, les fonctions et étapes décrites peuvent être mise en oeuvre sous forme de programme informatique ou via des composants matériels (p. ex. des réseaux de portes programmables). Les fonctions et étapes opérées par le système de pilotage (en particulier l'unité de traitement ou ses modules comme expliqué ci-après) peuvent être réalisées par des jeux d'instructions ou modules informatiques implémentés dans un processeur ou contrôleur ou être réalisées par des composants électroniques dédiés ou des composants de type FPGA ou ASIC. Il est aussi possible de combiner des parties informatiques et des parties électroniques.

Le système 4 de pilotage est ainsi un système électronique et/ou informatique. Lorsqu'il est précisé que le système ou des moyens ou module dudit système sont configurés pour réaliser une opération donnée, cela signifie que le système comprend des instructions informatiques et les moyens d'exécution correspondants qui permettent de réaliser ladite opération et/ou que le système comprend des composants électroniques correspondant.

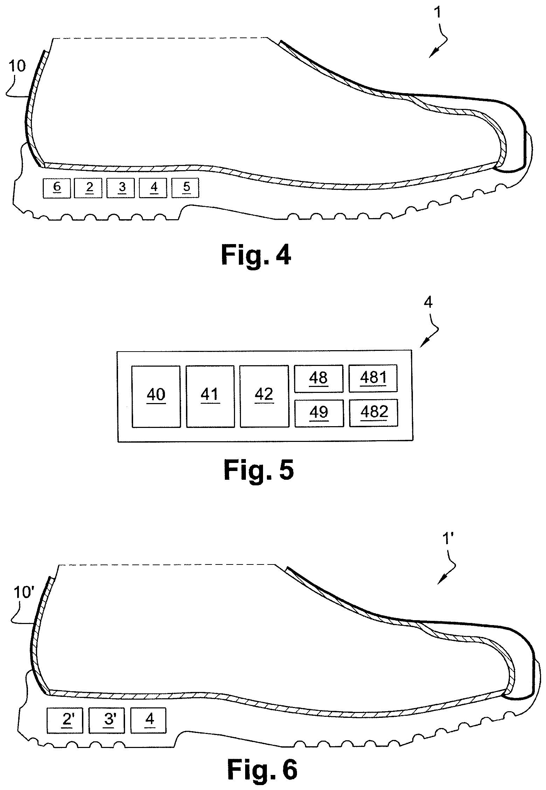

Le système de pilotage 4 comprend plusieurs composants. Un exemple schématique d'un tel système de pilotage 4 est illustré à la figure 5.

L'invention pouvant s'appliquer à une chaussure ou à une paire de chaussures, on comprend que les composants du système de pilotage 4 peuvent être disposés dans une seule chaussure ou être répartis dans deux chaussures comme dans l'exemple illustré aux figures 4 et 6. On comprend ainsi que les caractéristiques décrites dans le cas d'une chaussure sont aussi applicables au cas d'une paire de chaussures. En particulier, dans le cas d'une paire de chaussures, on peut prévoir comme illustré à la figure 6, que la deuxième chaussure 1' soit, comme la première chaussure 1, munie au niveau de son talon d'un accéléromètre 2' et d'un gyroscope 3', dont les données sont utilisables, en combinaison avec celles de l'accéléromètre 2 et du gyroscope 3 de la première chaussure 1 pour détecter la chute du porteur.

Selon un aspect particulier, les accéléromètres 2, 2' sont agencés de la même manière dans la première chaussure 1 et dans la deuxième chaussure 1'. On peut aussi prévoir que les gyroscopes 3, 3' soient agencés de la même manière dans la première chaussure 1' et dans la deuxième chaussure 1'.

Autrement dit, lorsque les chaussures sont orientées parallèlement l'une à l'autre, les axes de l'accéléromètre 2 sont parallèles aux axes de l'accéléromètre 2' et de même les axes du gyroscope 3 sont aussi parallèles aux axes du gyroscope 3'.

Le système de pilotage 4 comprend une mémoire 40 de données permettant d'enregistrer les données fournies par l'accéléromètre 2 et le gyroscope 3. Selon un aspect particulier, l'enregistrement est réalisé à une fréquence de 50 Hz.

La mémoire 40 dans laquelle sont enregistrées les données acquises peut être différente ou en tout ou partie commune avec la mémoire dans laquelle sont stockées les instructions informatiques.

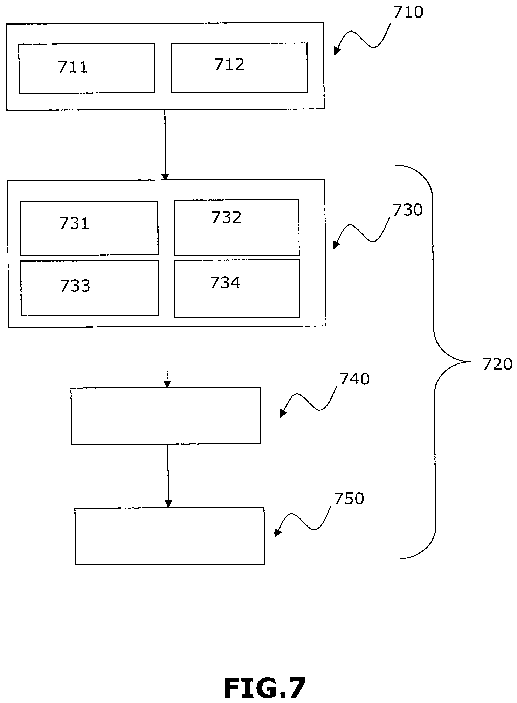

Le système de pilotage 4 comprend une unité de traitement 41, telle qu'un (micro)processeur informatique. Le système ou l'unité de traitement peut aussi être réalisé(e) sous la forme d'un microcontrôleur. L'unité de traitement 41 comprend un module de détection de chute configuré pour permettre d'exécuter un procédé de détection de chute 720 (voir figure 7) expliqué ci-après.

En cas de détection de chute, l'unité de traitement génère un signal correspondant de détection de chute. Ce signal de détection de chute est transmis par un module de communication 42 radio à un récepteur distant. Ledit récepteur est par exemple un numéro (ou identifiant) de téléphone d'une personne, encore appelée aidant, ou une liste de numéros identifiants de différentes personnes dont le numéro d'appel peut être prédéfini et/ou modifiable. On peut ainsi prévoir que la première personne reçoit un appel ou message correspondant à la détection de chute et en cas d'indisponibilité de cette première personne que l'appel ou message soit transmis au numéro suivant de la liste.

On peut prévoir que le porteur puisse infirmer le signal de détection de chute émis ou destiné à être émis, en effectuant par exemple une séquence prédéfinie de mouvements avec la ou chaque chaussure. Ce signal d'information peut lui aussi être transmis par le module de communication 42.

Selon un aspect particulier, ledit module de communication radio 42 est configuré pour communiquer selon au moins l'un des protocoles suivants, de type LPWAN : LORA ; Nb-iot ; LTE-M

Selon un mode de réalisation de l'invention, par exemple illustré dans le logigramme de la figure 7, l'exécution du procédé de détection de chute 720 est conditionnée au résultat d'un procédé de vérification 710 qui comprend une ou plusieurs étapes de vérification 711, 712. A cet effet, l'unité de traitement comprend un module de vérification configuré pour permettre d'exécuter ledit procédé de vérification 710.

Un mode de réalisation est présenté ci-après pour une chaussure, mais on peut prévoir que cette vérification soit effectuée pour chaque chaussure.

Selon une étape de vérification 711, l'unité de traitement vérifie si l'accélération, mesurée par l'accéléromètre sur au moins un axe, est supérieure à une valeur seuil, par exemple 6g, c'est-à-dire six fois l'accélération de la pesanteur à la surface de la Terre.

Selon une étape de vérification 712, l'unité de traitement vérifie que le pied est présent dans la chaussure. Selon un mode de réalisation, la chaussure comprend à cet effet un capteur 6 de présence de pied qui communique avec l'unité de traitement. Le capteur 6 de présence de pied est de préférence situé dans le talon de la chaussure.

Avantageusement, le capteur 6 de présence de pied est de type capacitif. L'utilisation d'un capteur capacitif permet de détecter la présence du pied dans la chaussure quelle que soit l'orientation du pied. En variante, le capteur de présence de pied peut être d'un autre type, par exemple un capteur d'effort.

La détection de la présence du pied dans la chaussure, permet de s'assurer que le processus de détection de chute soit déclenché seulement lorsque le pied de la personne est présent dans la chaussure et donc lorsque la personne est équipée de la chaussure et est potentiellement en situation de chute. L'utilisation d'un tel capteur permet ainsi d'éviter des détections intempestives assimilées à des chutes alors que la personne n'est pas équipée de sa chaussure.

On peut prévoir que lorsque le capteur n'émet plus de signal de présence de pied, pendant une durée donnée, par exemple correspondant à une temporisation, alors le système de pilotage se met en mode veille.

L'utilisation d'un capteur capacitif permet de conserver la détection d'un signal de présence de pied de manière fiable contrairement à un capteur d'effort qui risquerait de perdre le signal lorsque le pied est levé ou en fonction de l'orientation du pied.

Le déclenchement d'une des étapes de vérification peut être conditionné au résultat d'une ou de l'autre étape de vérification.

On peut ainsi prévoir que la présence du pied dans la chaussure est vérifiée (étape 712), si ladite accélération mesurée est supérieure à ladite valeur seuil (résultat possible de l'étape 711). Inversement, on peut prévoir de vérifier ladite accélération (étape 711), si le pied est présent dans la chaussure (résultat possible de l'étape 712).

Lorsque les conditions associées à la ou aux étapes de vérification 711, 712 du procédé de vérification 710 sont remplies, alors l'unité de traitement déclenche l'exécution du procédé de détection de chute 720. On peut ainsi prévoir que tant que le procédé de détection de chute 720 n'est pas déclenché, le système de pilotage 4 reste dans une configuration d'économie d'énergie, dont il sort lorsque les conditions associées à la ou aux étapes de vérification 711, 712 du procédé de vérification 710 sont remplies, afin d'exécuter le procédé de détection de chute 720.

Il est aussi prévu que lorsque les conditions du procédé de vérification 710 sont remplies, le procédé de détection de chute 720 est mis en oeuvre pour les données enregistrées sur une fenêtre de temps donnée, appelée fenêtre d'analyse. Les paramètres ou caractéristiques de définition de cette fenêtre d'analyse peuvent être fonctions des caractéristiques du procédé de vérification 710. Selon un aspect particulier, la fenêtre d'analyse correspond à une première durée donnée avant le temps auquel il est déterminé que l'accélération est supérieure à ladite valeur seuil donnée et à une deuxième durée donnée, qui peut ou non être différente de la première durée, après ce temps.

On peut ainsi prévoir que l'accéléromètre enregistre en continu les données d'accélération (cet enregistrement étant éventuellement conditionné à la détection de présence du pied dans la chaussure) et que le procédé de vérification s'effectue sur des données enregistrées pendant une fenêtre de temps donnée correspondant à ladite fenêtre d'analyse. Comme rappelé ci-dessus, les caractéristiques de ladite fenêtre d'analyse, telles que sa durée et sa position dans le temps, peuvent dépendre des caractéristiques associées au procédé de vérification, telles que l'instant où une ou plusieurs conditions dudit procédé de vérification ont été remplies en définissant une durée avant et une durée après cet instant.

Le procédé de détection de chute 720 comprend un procédé de détermination de paramètres de chute 730.

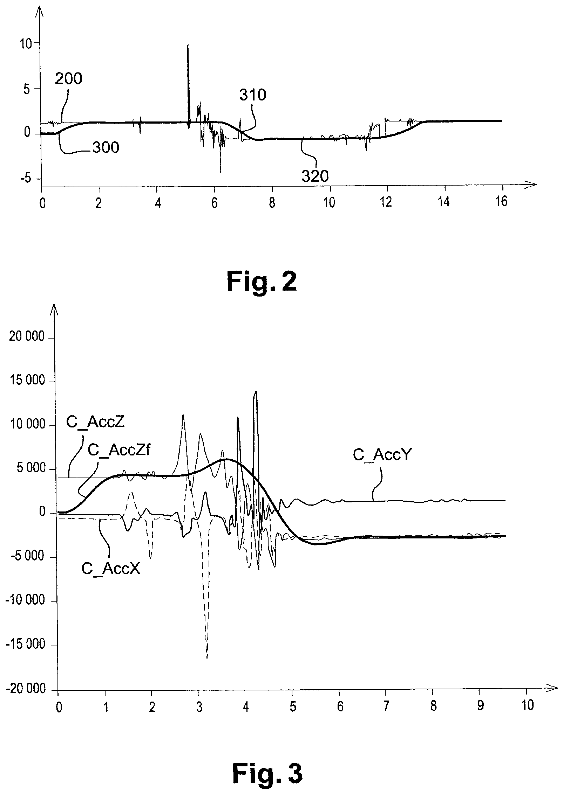

Le procédé de détermination de paramètres de chute 730 comprend une étape 731 de détermination de la pente de la courbe d'accélération selon l'axe Z en fonction du temps (notée C_AccZf dans l'exemple de la figure 3), la courbe étant de préférence filtrée. La pente de la courbe d'accélération est déterminée sur une plage de temps donnée qui correspond par exemple à la fenêtre de temps d'analyse ou une sous-fenêtre de temps dont le début peut par exemple correspondre à, ou être définie en fonction, de l'instant auquel l'accélération qui a servi au déclenchement du procédé de détection de chute, a été détectée.

Le filtrage permet de moyenner le signal afin d'obtenir une courbe plus lisse pour le calcul de la pente. Le filtre peut être un filtre numérique passe bas. On peut prévoir que la fréquence de coupure du filtre est inférieure à 0.5Hz, de sorte qu'un signal dont la fréquence est supérieure à 0.5Hz sera fortement atténué.

La pente permet d'analyser le changement de position du porteur depuis une position debout à une position allongée. La variation de la pente déterminée permet en particulier de déterminer le moment de la chute, qui peut correspondre à une forte valeur de pente descendante de l'accélération selon Z, et le moment qui suit où la personne tombée reste au sol qui peut être associé à une faible ou sensiblement nulle valeur de pente (ou encore inférieure à une valeur seuil), c'est-à-dire à une faible ou sensiblement nulle variation d'accélération selon l'axe Z de l'accéléromètre. La détermination de la pente de la courbe d'accélération selon l'axe Z permet ainsi de prendre en compte la valeur de la pente elle-même et/ou sa variation.

Il peut être déduit d'une pente importante dans le sens descendant que la personne est rapidement passée de la position verticale à une position horizontale. Il peut alors être considéré que l'événement associé est probablement une chute. Pour détecter la chute, on peut aussi prévoir de tenir compte de la baisse d'accélération sur l'axe Z combinée à une augmentation de l'accélération selon l'axe X ou Y.

Le procédé de détermination de paramètres de chute 730 comprend une étape 732 de détermination de la durée, dite durée de position horizontale, pendant laquelle l'accélération selon un axe, de préférence l'axe Z de l'accéléromètre, ou selon plusieurs des axes de l'accéléromètre, reste, de préférence de manière continue, inférieure à une valeur seuil.

Ladite valeur seuil peut être prédéfinie, ou fonction de la valeur d'accélération mesurée avant un temps donné dans la fenêtre d'analyse. On peut ainsi prévoir de considérer comme faisant partie de la durée de position horizontale, la durée pendant laquelle la valeur d'accélération mesurée après l'instant, encore appelé instant de référence, où une ou plusieurs conditions dudit procédé de vérification ont été remplies, est inférieure à une valeur seuil et/ou à la valeur d'accélération mesurée (sur un ou plusieurs axes) avant cet instant. Cet instant de référence utilisé pour la détermination de la durée de position horizontale peut aussi être un temps associé à une valeur de pente de la courbe d'accélération selon l'axe Z. On peut ainsi prévoir de déterminer cette durée à partir d'un temps correspondant à la fin d'une forte pente de baisse d'accélération selon l'axe Z.

La durée de position horizontale traduit un maintien de la personne en position horizontale. La durée de position horizontale peut aussi être comparée à une valeur seuil pour par exemple pondérer sa prise en compte dans la détection de chute et en outre pour déterminer si la personne est ou non consciente.

On peut prévoir en particulier que le module de traitement 41 analyse les variations d'accélération mesurées selon un ou plusieurs axes de l'accéléromètre 2 pendant une durée donnée de position horizontale, pour déterminer si le porteur de la chaussure est ou non conscient et émettre un signal adapté. Il est aussi possible en fonction de niveaux de seuils prédéfinis de différencier une action de chute volontaire d'une action de chute involontaire.

Comme rappelé ci-dessus, on peut prévoir que la durée de position horizontale soit déterminée à partir d'un temps donné de la fenêtre de temps, qui correspond par exemple à l'instant où une ou plusieurs conditions dudit procédé de vérification (dont l'issue positive déclenche le procédé de détection de chute) ont été remplies.

Les étapes 731 et 732 peuvent être exécutées en parallèle ou séquentiellement dans un sens ou dans l'autre. L'exécution de ces étapes peuvent être conditionnées l'une au résultat de l'autre.

Le procédé de détermination de paramètres de chute 730 peut aussi comprendre une étape 733 de détermination de la valeur d'accélération maximale détectée pour un axe donné, ou pour chaque axe d'une pluralité d'axes de l'accéléromètre, de préférence pour chaque axe X, Y, Z de l'accéléromètre. Selon un aspect particulier la valeur d'accélération maximale détectée peut être la valeur de choc maximale parmi les chocs détectés (comme expliqué ci-après).

Le procédé de détermination de paramètres de chute 730 peut aussi comprendre une étape 734 de détermination du nombre de chocs détectés. On peut considérer qu'un choc correspond à une accélération selon au moins l'un des axes de l'accéléromètre, supérieure à une valeur seuil donnée, telle que 3g c'est-à-dire trois fois l'accélération de la pesanteur à la surface de la Terre. On peut prévoir de ne tenir compte que des chocs associés à un axe donné ou associés à plusieurs des axes de l'accéléromètre ou encore de prendre les chocs associés à chaque axe.

Ces étapes 733, 734 peuvent, comme pour les étapes 731, 732 être menées en parallèle ou séquentiellement, en conditionnant éventuellement leur exécution au résultat de l'une ou l'autre desdites étapes.

Selon un autre aspect particulier, on peut aussi prévoir de déterminer comme paramètre de chute, la vitesse de giration autour de l'axe X et/ou Y mesurée à l'aide du gyroscope lorsqu'il est présent, ce qui permet d'analyser la vitesse de chute. La valeur de vitesse de giration peut aussi être comparée à une valeur seuil pour par exemple pondérer sa prise en compte dans la détection de chute. En particulier, le gyroscope permet de mesurer une vitesse de rotation (exprimée en radian par seconde) autour des axes X et Y. Une vitesse de rotation mesurée importante permet de déterminer que le pied correspondant de la personne a tourné rapidement, ce qui peut être représentatif d'une chute de la personne. Cette vitesse de rotation peut aussi être déterminée à l'aide des données d'accéléromètre.

Selon un mode de réalisation préféré, le procédé de détermination de paramètres de chute 730 est suivi d'une étape de détermination d'orientation 740 de la chaussure selon laquelle l'unité de traitement détermine une donnée représentative de l'orientation de la chaussure par rapport à la verticale.

Cette étape de détermination d'orientation 740 permet de déterminer si la chaussure est en position dite « droite » ou « verticale », correspondant à un état sensiblement debout de la personne équipée de la chaussure ou si la chaussure est sensiblement à l'horizontal, correspondant à un état allongé de la personne.

En particulier, on peut prévoir que l'unité de traitement détermine que la chaussure est en position « droite » lorsque l'accélération mesurée sur l'axe Z dépasse, pendant une durée donnée (temporisation), est dans une plage de valeurs par exemple autour de 1g, correspondant à la gravité, tandis que, éventuellement, l'accélération mesurée sur les autres axes X et/ou Y reste en dehors de cette plage.

Cette détermination de l'orientation ou position de la chaussure peut s'effectuer sur une fenêtre de temps correspondant par exemple à une sous-fenêtre de ladite fenêtre d'analyse. On peut prévoir que cette étape 740 soit réalisée sur une durée correspondant à la fin de la fenêtre d'analyse, par exemple sur une fenêtre identique ou similaire à celle pour laquelle la durée de position horizontale est déterminée.

Le procédé de détection de chute 720 comprend aussi une étape de détection de chute 750 qui émet un signal de détection de chute en fonction des paramètres de chute, et de préférence du paramètre d'orientation, précédemment déterminés.

Selon un mode de réalisation particulier, l'unité de traitement affecte des poids aux paramètres de chute et d'orientation déterminés. Les différents paramètres utilisés pour la détection de chute peuvent ainsi être pondérés les uns par rapport aux autres.

Le poids associé à un paramètre peut être fonction du type de paramètre et/ou de sa valeur même (en la comparant à une valeur seuil).

L'unité de traitement 41 calcule alors une valeur, appelée score, en fonction desdits paramètres déterminés, de préférence pondérés.

On peut aussi prévoir que le score soit pondéré par exemple en fonction du poids et/ou de la taille de la personne. Ces facteurs de pondération peuvent être entrés au moyen d'une interface adaptée de communications avec le système de pilotage 4.

Selon un aspect particulier, l'unité de traitement 41 détecte que ledit score correspond à une chute lorsque la valeur du score est supérieure à une valeur seuil prédéfinie. Le signal de détection de chute peut alors être généré et émis comme rappelé ci-dessus.

Selon un mode de réalisation, la chaussure 1 comprend aussi un module de géolocalisation raccordé de manière directe ou indirecte à l'unité de traitement 41 pour permettre d'acquérir la position géographique de la chaussure et transmettre cette position par ledit module de communication 42 à un récepteur distant. Préférentiellement, l'activation du module de géolocalisation est asservie à la détection d'une chute, éventuellement après une durée donnée faisant suite à la détection de chute pour permettre au porteur d'invalider la détection de chute. Un tel pilotage de l'activation du module de géolocalisation permet d'économiser l'énergie et de n'envoyer le signal de géolocalisation qu'en cas de chute avérée.

Préférentiellement, le talon de la chaussure 1 comprend aussi un vibreur 5, qui est raccordé à l'unité de traitement 41. L'activation du vibreur est aussi commandée par l'unité de traitement 41. On peut en particulier prévoir que le vibreur serve de moyens de communication (confirmation ou avertissement) sensoriel par exemple pour permettre à un aidant de confirmer au porteur qu'il a reçu le signal de détection de chute ou encore pour avertir qu'un signal de détection de chute va être émis.

La chaussure 1 peut aussi être munie d'un élément lumineux, tel qu'une LED 481 ou 482, raccordée à l'unité de traitement 41 et qui peut, de manière similaire au vibreur, donner des informations au porteur, par exemple des informations d'accusé de réception ou d'alerte.

Selon un aspect particulier, les composants électroniques inclus dans le talon sont logés dans un même boîtier, de préférence noyé dans la matière du talon.

Avantageusement, les composants intégrés dans le talon peuvent être logés dans un boîtier nervuré ou de manière générale présentant des éléments de rugosité pour adhérer à la matière, par exemple du Polyuréthane, dans laquelle la semelle, et notamment le talon est réalisé, et dans laquelle ledit boîtier est intégré.

Dans le cas d'un mode de réalisation prévoyant de répartir le système de pilotage dans deux chaussures 1,1' comme illustré aux figures 4 et 6, chacune des chaussures 1,1' comprend un module de communication 48, 49 radio, de préférence selon le protocole Bluetooth.

Lesdits modules de communication 48, 49 peuvent communiquer entre eux pour transmettre des données de composants, tels que les données d'accéléromètre 2' et de gyroscope 3', depuis le module de communication 49 de la deuxième chaussure 1' à l'unité de traitement41 de la première chaussure 1. On peut aussi prévoir que le module de communication 48 puisse transmettre des données de la première chaussure 1 au module de communication 49 de la deuxième chaussure 1'.

La figure 2 est un graphique sur lequel sont tracées en fonction du temps, un exemple de courbe 200 de l'accélération mesurée selon l'axe Z, ainsi que la courbe 300 de l'accélération mesurée selon l'axe Z et filtrée.

Comme illustré à la figure 2, la courbe présente dans sa portion 310 une pente qui est représentative d'une chute potentielle du porteur. Comme rappelé ci-dessus, l'unité de traitement 41 permet de détecter une telle pente et d'en tenir compte pour déterminer si une chute s'est produite.

Selon un aspect particulier, l'unité de traitement analyse les données de la courbe et/ou des courbes d'accélération selon chacun des axes X, Y, Z, ou selon une partie de ces axes, avant, pendant et/ou après cette pente pour affiner l'analyse de détection de chute. L'unité de traitement permet en particulier d'analyser les variations d'accélération mesurées selon au moins l'un des axes, par exemple l'axe Z, pendant une durée donnée de position horizontale. En particulier, le module de traitement analyse les variations d'accélération selon l'axe Z filtrées sur une durée donnée, par exemple sur la portion 320 de la courbe 300 en aval de la portion 310 correspondant à une détection du fait que la pente de la courbe a dépassé une valeur seuil donnée sur une durée donnée. Cette analyse des vibrations en aval, permet d'identifier si le porteur remue un peu ou non alors qu'il est en position horizontale, ce qui permet en cas de détection de mouvement suffisant de considérer que le porteur est allongé mais conscient.

La Figure 3 est un graphique sur lequel est tracé en fonction du temps différentes courbes d'accélérations mesurées sur une période de temps donnée pendant laquelle une personne munie de la ou de chaque chaussure trébuche et tombe. Lesdites courbes sont ainsi superposées et comprennent les courbes C_ AccZ, C_ AccX, C_ AccY de l'accélération mesurée selon respectivement l'axe Z, l'axe X et l'axe Y. Lesdites courbes superposées comprennent aussi la courbe C_ AccZf qui est la courbe de l'accélération mesurée selon l'axe Z et filtrée pour pouvoir déterminer dans la courbe une pente représentative de la chute. On constate en particulier que, dans l'intervalle 2,5 - 5 secondes, la pente augmente puis descend rapidement jusqu'à reprendre une valeur moyenne sensiblement nulle (ce qui peut être détecté par la détermination de la durée de position horizontale sur une fenêtre définie après la pente rapide). On peut observer que dans la zone de temps correspondant à la variation de pente importante, de nombreux pics d'amplitudes apparaissent pour chaque courbe, ce que l'unité de traitement 41 peut analyser pour identifier ou confirmer un cas de chute.

L'invention n'est pas limitée aux modes de réalisation illustrés dans les dessins.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Chaussure (1) comprenant un accéléromètre (2) permettant de fournir des données d'accélération (AccX, AccY, AccZ) selon trois axes distincts X, Y, Z, l'axe Z étant orienté verticalement lorsque la chaussure (1) est posée à plat sur un sol horizontal,
ladite chaussure (1) comportant aussi un système de pilotage (4) qui comprend :
- une mémoire (40) permettant d'enregistrer les données fournies par l'accéléromètre (2);
- une unité de traitement (41) de données, telle qu'un processeur, l'unité de traitement (41) étant configurée pour réaliser
les étapes suivantes :
- comparer l'accélération mesurée par l'accéléromètre selon au moins un axe, avec une valeur seuil, telle que 6g, et,
- en fonction au moins du résultat de la comparaison, déclencher la détermination des paramètres suivants :
- la pente de la courbe (C_AccZf), de préférence filtrée, de l'accélération mesurée selon l'axe Z en fonction du temps,
- la durée, dite durée de position horizontale, pendant laquelle l'accélération ou la variation d'accélération selon un axe (Z), ou plusieurs des axes (X,Y,Z) de l'accéléromètre, reste inférieure à une valeur seuil;
- détecter une chute au moins en fonction de ladite pente et de ladite durée de position horizontale,
ledit module de traitement (41) étant configuré pour, en cas de détection de chute, générer un signal correspondant de détection de chute ;
et en ce que le système de pilotage (4) comprend aussi un module de communication (42) radio permettant de transmettre ledit signal de détection de chute à un récepteur distant.

2. Chaussure (1) selon la revendication 1, dans laquelle l'unité de traitement (41) est configurée pour réaliser ladite détermination de paramètres à partir des données enregistrées sur une fenêtre de temps donnée, ladite fenêtre étant définie par une première durée donnée avant l'instant auquel l'unité de traitement (41) détermine que ladite accélération mesurée est supérieure à ladite valeur seuil donnée, et par une deuxième durée donnée après cet instant.

3. Chaussure (1) selon la revendication 2, dans laquelle l'unité de traitement (41) est configurée pour déterminer ladite durée de position horizontale sur une partie de ladite fenêtre de temps dont l'instant de début est défini en fonction d'au moins l'un des paramètres suivants :
- la pente déterminée,
- l'instant auquel l'unité de traitement (41) détermine que l'accélération mesurée est supérieure à ladite valeur seuil donnée.

4. Chaussure (1) selon l'une quelconque des revendications précédentes, dans laquelle l'unité de traitement (41) est aussi configurée pour déterminer pour au moins un, de préférence pour chaque, axe (X, Y, Z) de l'accéléromètre, une valeur d'accélération maximale, et pour détecter la chute en fonction aussi de ladite valeur d'accélération maximale.

5. Chaussure (1) selon l'une quelconque des revendications précédentes, dans laquelle l'unité de de traitement (41) est aussi configurée pour déterminer un nombre de chocs, un choc correspondant à une accélération selon au moins l'un des axes de l'accéléromètre, supérieure à une valeur seuil données, telle que 3g, et pour détecter la chute en fonction aussi dudit nombre de chocs.

6. Chaussure (1) selon l'une quelconque des revendications précédentes, dans laquelle l'unité de traitement (41) est aussi configurée pour déterminer l'orientation de la chaussure par rapport à la verticale, et pour détecter la chute en fonction aussi de ladite orientation de la chaussure.

7. Chaussure (1) selon l'une quelconque des revendications précédentes, dans laquelle la chaussure comprend un gyroscope (3) permettant de fournir des données de giration au moins autour de l'axe X et/ou Y, et l'unité de traitement (41) est configurée pour détecter la chute en fonction aussi des données de giration.

8. Chaussure (1) selon l'une quelconque des revendications précédentes, dans laquelle la chaussure comprend un capteur (6) de détection de la présence d'un pied dans la chaussure.

9. Chaussure (1) selon la revendication 8, dans laquelle le capteur (6) est un capteur capacitif, de préférence logé dans un talon de la chaussure.

10. Chaussure (1) selon l'une quelconque des revendications 8 ou 9, dans laquelle l'unité de traitement (41) comprend aussi un module de vérification configuré pour détecter (712) la présence d'un pied d'une personne dans la chaussure, et le déclenchement de la détermination (730) de paramètres est aussi fonction du résultat de la détection (712) de présence de pied dans la chaussure.

11. Chaussure (1) selon l'une quelconque des revendications précédentes, dans laquelle la chaussure (1) présente un talon (10) dans lequel l'accéléromètre (2) est logé.

12. Chaussure (1) selon l'une quelconque des revendications précédentes, dans laquelle la chaussure (1) comprend un module de géolocalisation raccordé de manière directe ou indirecte au système de pilotage (4) pour permettre d'acquérir la position géographique de la chaussure et transmettre cette position par ledit module de communication (42) à un récepteur distant.

13. Chaussure (1) selon l'une quelconque des revendications précédentes, dans laquelle la chaussure (1) comprend aussi un vibreur (5), de préférence inclus dans un talon (10) de la chaussure, et raccordé de manière directe ou indirecte au système de pilotage (4).

14. Ensemble comprenant une première chaussure (1) selon l'une quelconque des revendications précédentes, et
une deuxième chaussure (1') comprenant un accéléromètre (2') de préférence inclus dans un talon (10') de la deuxième chaussure,
dans lequel chacune des première et deuxième chaussures (1, 1') comprend un module de communication (48, 49) radio, de préférence selon le protocole Bluetooth,
lesdits modules de communication (48, 49) étant aptes à communiquer entre eux pour transmettre les données d'accéléromètre (2'), depuis le module de communication (49) de la deuxième chaussure (1') au module de traitement (41) de la première chaussure (1).

15. Procédé de détection de chute à l'aide d'une chaussure (1) selon l'une quelconque des revendications 1 à 13 ou d'un ensemble de chaussures (1, 1') selon la revendication 14,
dans lequel le procédé de détection de chute comprend les étapes suivantes :
- comparer (711) l'accélération mesurée par l'accéléromètre selon au moins un axe, avec une valeur seuil, et,
- en fonction au moins du résultat de la comparaison, déclencher un procédé de détermination (730) de paramètres qui comprend les étapes suivantes :
- détermination (731) de la pente de la courbe (C_AccZf), de préférence filtrée, de l'accélération mesurée selon l'axe Z en fonction du temps,
- détermination (732) de la durée, dite durée de position horizontale, pendant laquelle l'accélération ou la variation d'accélération selon un axe (Z) ou plusieurs des axes (X,Y,Z) de l'accéléromètre, reste inférieure à une valeur seuil ;
- détecter (750) une chute au moins en fonction de ladite pente et de ladite durée de position horizontale,
et le procédé comprenant la génération d'un signal correspondant de détection de chute, et la transmission dudit signal de détection de chute à un récepteur distant.

16. Produit de programme informatique non transitoire comprenant des instructions de code de programme pour l'exécution des étapes du procédé de détection de chute de la revendication 15, à l'aide d'une chaussure (1) selon l'une quelconque des revendications 1 à 13 ou d'un ensemble de chaussures (1, 1') selon la revendication 14, lorsque ledit programme est exécuté par un processeur de la ou d'une des chaussures.

## Patentansprüche

1. Schuh (1) mit einem Beschleunigungsmesser (2), der ermöglicht, Beschleunigungsdaten (AccX, AccY, AccZ) gemäß drei verschiedenen Achsen X, Y, Z zu liefern, wobei die Achse Z vertikal ausgerichtet ist, wenn der Schuh (1) eben auf einen horizontalen Boden gestellt ist, wobei der Schuh (1) auch ein Steuersystem (4) umfasst, das Folgendes umfasst:
- einen Speicher (40), der ermöglicht, die Daten aufzuzeichnen, die vom Beschleunigungsmesser (2) geliefert werden;
- eine Datenverarbeitungseinheit (41) wie z. B. einen Prozessor, wobei die Datenverarbeitungseinheit (41) konfiguriert ist, um die folgenden Schritte durchzuführen:
- Vergleichen der Beschleunigung, gemessen vom Beschleunigungsmesser gemäß mindestens einer Achse, mit einem Schwellenwert, wie z. B. 6g, und
- in Funktion mindestens des Ergebnisses des Vergleichs, Auslösen der Bestimmung der folgenden Parameter:
-- des Gefälles der Kurve (C_AccZf), vorzugsweise gefiltert, der Beschleunigung, gemessen gemäß der Achse Z in Funktion der Zeit,
-- der Dauer, bezeichnet als Dauer der horizontalen Position, während der die Beschleunigung oder die Variation der Beschleunigung gemäß einer Achse (Z) oder mehrerer der Achsen (X, Y, Z) des Beschleunigungsmessers kleiner als ein Schwellenwert bleibt;
- Erfassen eines Falls, mindestens in Funktion des Gefälles und der Dauer der horizontalen Position,
wobei das Verarbeitungsmodul (41) konfiguriert ist, um bei der Erfassung eines Falls ein entsprechendes Signal zur Erfassung eines Falls zu generieren;
und dadurch, dass das Führungssystem (4) auch ein Funkkommunikationsmodul (42) umfasst, das ermöglicht, das Fallerfassungssignal an einen entfernten Empfänger zu übertragen.

2. Schuh (1) nach Anspruch 1, wobei die Verarbeitungseinheit (41) konfiguriert ist, um die Bestimmung von Parametern ausgehend von Daten durchzuführen, die in einem bestimmten Zeitfenster aufgezeichnet wurden, wobei das Fenster durch eine erste bestimmte Dauer vor dem Zeitpunkt, an dem die Verarbeitungseinheit (41) bestimmt, dass die gemessene Beschleunigung grösser als der bestimmte Schwellenwert ist, und durch eine zweite bestimmte Dauer nach diesem Zeitpunkt definiert ist.

3. Schuh (1) nach Anspruch 2, wobei die Verarbeitungseinheit (41) konfiguriert ist, um die Dauer der horizontalen Position in einem Teil des Zeitfensters zu bestimmen, dessen Anfangszeitpunkt in Funktion mindestens eines der folgenden Parameter definiert ist:
- des vorbestimmten Gefälles,
- des Zeitpunkts, an dem die Verarbeitungseinheit (41) bestimmt, dass die gemessene Beschleunigung grösser als der bestimmte Schwellenwert ist.

4. Schuh (1) nach einem der vorhergehenden Ansprüche, wobei die Verarbeitungseinheit (41) auch konfiguriert ist, um für mindestens eine, vorzugsweise für jede Achse (X, Y, Z) des Beschleunigungsmessers einen maximalen Beschleunigungswert zu bestimmen, und um den Fall auch in Funktion des maximalen Beschleunigungswerts zu erfassen.

5. Schuh (1) nach einem der vorhergehenden Ansprüche, wobei die Verarbeitungseinheit (41) auch konfiguriert ist, um eine Anzahl von Stößen zu bestimmen, wobei ein Stoß einer Beschleunigung gemäß mindestens einer der Achsen des Beschleunigungsmessers entspricht, die grösser als ein bestimmter Schwellenwert ist, wie z. B. 3g, und um den Fall in Funktion auch der Anzahl von Stößen zu erfassen.

6. Schuh (1) nach einem der vorhergehenden Ansprüche, wobei die Verarbeitungseinheit (41) auch konfiguriert ist, um die Ausrichtung des Schuhs mit Bezug auf die Vertikale zu bestimmen, und um den Fall in Funktion auch der Ausrichtung des Schuhs zu erfassen.

7. Schuh (1) nach einem der vorhergehenden Ansprüche, wobei der Schuh ein Gyroskop (3) umfasst, das ermöglicht, Daten der Drehung mindestens um die Achse X und/oder Y zu liefern, und die Verarbeitungseinheit (41) konfiguriert ist, um den Fall in Funktion auch der Drehung zu erfassen.

8. Schuh (1) nach einem der vorhergehenden Ansprüche, wobei der Schuh einen Sensor (6) zum Erfassen der Anwesenheit eines Fußes im Schuh umfasst.

9. Schuh (1) nach Anspruch 8, wobei der Sensor (6) ein kapazitiver Sensor ist, der vorzugsweise in einer Ferse des Schuhs untergebracht ist.

10. Schuh (1) nach einem der Ansprüche 8 oder 9, wobei die Verarbeitungseinheit (41) auch ein Überprüfungsmodul umfasst, das konfiguriert ist, um die Anwesenheit eines Fußes einer Person in einem Schuh zu erfassen (712) und die Auslösung der Bestimmung (730) von Parametern auch eine Funktion des Ergebnisses der Erfassung (712) der Anwesenheit eines Fußes im Schuh ist.

11. Schuh (1) nach einem der vorhergehenden Ansprüche, wobei der Schuh (1) eine Ferse (10) aufweist, in der der Beschleunigungsmesser (2) untergebracht ist.

12. Schuh (1) nach einem der vorhergehenden Ansprüche, wobei der Schuh (1) ein Geolokalisierungsmodul umfasst, das auf direkte oder indirekte Weise mit dem Führungssystem (4) verbunden ist, um zu ermöglichen, die geographische Position des Schuhs zu erhalten und diese Position durch das Kommunikationsmodul (42) an einen entfernten Empfänger zu übertragen.

13. Schuh (1) nach einem der vorhergehenden Ansprüche, wobei der Schuh (1) auch einen Vibrator (5) umfasst, der vorzugsweise in einer Ferse (10) des Schuhs eingeschlossen und auf direkte oder indirekte Weise mit dem Führungssystem (4) verbunden ist.

14. Anordnung, umfassend einen ersten Schuh (1) nach einem der vorhergehenden Ansprüche und einen zweiten Schuh (1'), umfassend einen Beschleunigungsmesser (2), der vorzugsweise in einer Ferse (10') des zweiten Schuhs eingeschlossen ist, wobei jeder des ersten und des zweiten Schuhs (1, 1') ein Funkkommunikationsmodul (48, 49) umfasst, vorzugsweise gemäß dem Bluetooth-Protokoll, wobei die Kommunikationsmodule (48, 49) ausgelegt sind, um untereinander zu kommunizieren, um die Daten des Beschleunigungsmessers (2') vom Kommunikationsmodul (49) des zweiten Schuhs (1') an das Verarbeitungsmodul (41) des ersten Schuhs (1) zu übertragen.

15. Fallerfassungsverfahren mit Hilfe eines Schuhs (1) nach einem der Ansprüche 1 bis 13 oder einer Anordnung von Schuhen (1, 1') nach Anspruch 14, wobei das Fallerfassungsverfahren die folgenden Schritte umfasst:
- Vergleichen (711) der Beschleunigung, gemessen vom Beschleunigungsmesser gemäß mindestens einer Achse, mit einem Schwellenwert, und
- in Funktion mindestens des Ergebnisses des Vergleichs, Auslösen eines Verfahrens zur Bestimmung (730) von Parametern, das die folgenden Schritte umfasst:
-- Bestimmen (731) des Gefälles der Kurve (C_AccZf), vorzugsweise gefiltert, der Beschleunigung, gemessen gemäß der Achse Z in Funktion der Zeit,
-- Bestimmen (732) der Dauer, bezeichnet als Dauer der horizontalen Position, während der die Beschleunigung oder die Variation der Beschleunigung gemäß einer Achse (Z) oder mehrerer der Achsen (X, Y, Z) des Beschleunigungsmessers kleiner als ein Schwellenwert bleibt;
- Erfassen (750) eines Falls, mindestens in Funktion des Gefälles und der Dauer der horizontalen Position,
und wobei das Verfahren das Erzeugen eines entsprechenden Fallerfassungssignals und das Übertragen des Fallerfassungssignals an einen entfernten Empfänger umfasst.

16. Nichttransitorisches Computerprogrammprodukt, umfassend Programmcodeanweisungen für die Durchführung der Schritte des Fallerfassungsverfahrens von Anspruch 15 mit Hilfe eines Schuhs (1) nach einem der Ansprüche 1 bis 13 oder einer Anordnung von Schuhen (1, 1') nach Anspruch 14, wenn das Programm von einem Prozessor des oder eines der Schuhe durchgeführt wird.

## Claims

1. A shoe (1) comprising an accelerometer (2) making it possible to supply acceleration data (AccX, AccY, AccZ) along three distinct axes X, Y, Z, the axis Z being oriented vertically when the shoe (1) is placed flat on a horizontal ground,
said shoe (1) also comprising a control system (4) which comprises:
- a memory (40) making it possible to record the data supplied by the accelerometer (2);
- a data processing unit (41), such as a processor,
the processing unit (41) being configured to carry out the following steps:
- comparing the acceleration measured by the accelerometer along at least one axis, with a threshold value, such as 6 g, and
- based on at least the result of the comparison, triggering the determination of the following parameters:
- the slope of the curve (C_AccZf), preferably filtered, of the acceleration measured along the axis Z as a function of time,
- the duration, called horizontal position duration, during which the acceleration or the acceleration variation along an axis (Z), or several of the axes (X, Y, Z), of the accelerometer remains below a threshold value;
- detecting a drop at least based on said slope and said horizontal position duration,
said processing module (41) being configured, if a drop is detected, to generate a corresponding drop detection signal;
and in that the control system (4) also comprises a radiocommunication module (42) making it possible to transmit said drop detection signal to a remote receiver.

2. The shoe (1) according to claim 1, wherein the processing unit (41) is configured to perform said parameter determination from data recorded over a given time window, said window being defined by a first given duration before the instant at which the processing unit (41) determines that said measured acceleration is greater than said given threshold value, and by a second given duration after this instant.

3. The shoe (1) according to claim 2, wherein the processing unit (41) is configured to determine said horizontal position duration over a part of said time window whose start instant is defined based on at least one of the following parameters:
- the determined slope,
- the instant at which the processing unit (41) determines that the measured acceleration is greater than said given threshold value.

4. The shoe (1) according to any one of the preceding claims, wherein the processing unit (41) is also configured to determine, for at least one, preferably for each, axis (X, Y, Z) of the accelerometer, a maximum acceleration value, and to detect the drop also based on said maximum acceleration value.

5. The shoe (1) according to any one of the preceding claims, wherein the processing unit (41) is also configured to determine a number of impacts, an impact corresponding to an acceleration along at least one of the axes of the accelerometer, greater than a given threshold value, such as 3 g, and to detect the drop also according to said number of impacts.

6. The shoe (1) according to any one of the preceding claims, wherein the processing unit (41) is also configured to determine the orientation of the shoe relative to the vertical plane, and to detect the drop also according to said orientation of the shoe.

7. The shoe (1) according to any one of the preceding claims, wherein the shoe comprises a gyroscope (3) making it possible to supply gyration data at least about the axis X and/or Y, and the processing unit (41) is configured to detect the drop also according to the gyration data.

8. The shoe (1) according to any one of the preceding claims, wherein the shoe comprises a sensor (6) for detecting the presence of a foot in the shoe.

9. The shoe (1) according to claim 8, wherein the sensor (6) is a capacitive sensor, preferably housed in a heel of the shoe.

10. The shoe (1) according to any one of claims 8 or 9, wherein the processing unit (41) also comprises a verification module configured to detect (712) the presence of a foot of a person in the shoe, and the triggering of the determination (730) of parameters also depends on the result of the detection (712) of the presence of a foot in the shoe.

11. The shoe (1) according to any one of the preceding claims, wherein the shoe (1) has a heel (10) wherein the accelerometer (2) is housed.

12. The shoe (1) according to any one of the preceding claims, wherein the shoe (1) comprises a geolocation module connected directly or indirectly to the control system (4) to make it possible to acquire the geographical position of the shoe and to transmit this position via said communication module (42) to a remote receiver.

13. The shoe (1) according to any one of the preceding claims, wherein the shoe (1) also comprises a vibrating unit (5), preferably included in a heel (10) of the shoe, and connected directly or indirectly to the control system (4).

14. An assembly comprising a first shoe (1) according to any one of the preceding claims, and
a second shoe (1') comprising an accelerometer (2') preferably included in a heel (10') of the second shoe,
wherein each of the first and second shoes (1, 1') comprises a radiocommunication module (48, 49), preferably according to the Bluetooth protocol,
said communication modules (48, 49) being able to communicate with one another to transmit the accelerometer data (2'), from the communication module (49) of the second shoe (1') to the processing module (41) of the first shoe (1).

15. A method for detecting a fall using a shoe (1) according to any one of claims 1 to 13 or a set of shoes (1, 1') according to claim 14,
wherein the fall detection method comprises the following steps:
- comparing (711) the acceleration measured by the accelerometer along at least one axis with a threshold value, and
- based on at least the result of the comparison, triggering a method for determining (730) parameters which comprises the following steps:
- determining (731) the slope of the curve (C_AccZf), preferably filtered, of the acceleration measured along the axis Z as a function of time,
- determining (732) the duration, called horizontal position duration, during which the acceleration or the acceleration variation along an axis (Z) or several of the axes (X, Y, Z) of the accelerometer remains below a threshold value;
- detecting (750) a fall at least based on said slope and said horizontal position duration,
and the method comprising generating a corresponding fall detection signal, and transmitting said fall detection signal to a remote receiver.

16. A non-transient computer program product comprising program code instructions for carrying out the steps of the fall detection method of claim 15, using a shoe (1) according to any one of claims 1 to 13 or a set of shoes (1, 1') according to claim 14, when said program is executed by a processor of the or one of the shoes.
